# EUROPEAN PATENT SPECIFICATION

(11) **EP 2 821 019 B1**
(45) Date of publication and mention of the grant of the patent: **01.02.2017**
(21) Application number: 14187573.2
(22) Date of filing: 05.06.2009
(51) Int. Cl.: A61B 17/56, A61F 2/46, A61M 25/00, A61B 17/88, A61B 17/70

(54) **A filler for spine**
Füller für der Wirbelsäule
Remplisseur pour la colonne vertébrale

(43) Date of publication of application: 07.01.2015
(62) Divisional of application: 09845374.9
(73) Proprietor: Shao, Weixing, Shandong 250014 (CN)
(72) Inventor: Shao, Weixing, Shandong 250014 (CN)
(74) Representative: Sun, Yiming

(56) References cited:
- CN-C- 100 389 728
- CN-Y- 2 840 933
- US-A1- 2007 255 281
- US-A1- 2007 270 876
- US-B1- 6 248 110

## Description

### Field of the Invention

The present invention relates to a kind of medical device, especially a filler for spine which is suit for comminuted fracture.

### Description of the Related Art

The widely-known technology is disclosed in the Chinese patent application NO. 200820173700.0 (CN201282981) from the present applicant in the existing technologies, the patent discloses a spreader and filler for surgery of compression fracture, this filler includes catheter and catheter holder for fastening catheter, there is a guide pin inside of the catheter, and the guide pin is fixed on the guide pin holder, a feed bag is set at one end of the catheter, the joint of the said feed bad connects to the catheter with thread, revolving catheter must be done for the separation of feed bag and catheter of this kind of connection after surgery finished, that increases the difficulty of the surgery, in addition, the joint should be left in the body. Those are the shortcomings of the existing technologies.

The patent US 6,248,110 B1 discloses systems and methods, which treat fractured or diseased bone by deploying more than a single therapeutic tool into the bone. In one arrangement, the systems and methods deploy an expandable body in association with a bone cement nozzle into the bone, such that both occupy the bone interior at the same time. In another arrangement, the systems and methods deploy multiple expandable bodies, which occupy the bone interior volume simultaneously. Expansion of the bodies form cavity or cavities in cancellous bone in the interior bone volume.

The patent application US 2007/0255281 A1 discloses a method, which is provided for inserting an instrument set having a tapered configuration into a patient. Furthermore, a method provided for inserting a cannula having an external threaded portion into bone is disclosed. In yet another preferred aspect, a method is provided for forming and enlarging an opening in bone.

The patent application US 2007/0270876 A1 discloses a vertebra bone cement introduction system, which includes a main frame comprised of lateral handle and vertical insertion tube; a pouring frame comprised of lateral handle and vertical extension tube; an external injection device to inject cement into extension tube; a taper being meshed to extension tube; a polygonal hole disposed to taper and a recession in the mid of taper connected to a silicon gel bag containing cement; a locking rod penetrating the extension tube; and the lateral rod of the pouring frame being turned in reverse to disengage taper from extension tube while leaving taper and bag in a hole drilled on the bone.

### Summary of the invention

The purpose of the present invention is to offer a technical embodiment of a filler for spine aiming at the shortcomings of existing technologies. The feed bag is set at the outer wall of the catheter by outer thimble in the programs, which is convenient for surgery, when the surgery finished, the catheter and outer thimble could be drawn out and useless foreign matter need not leave in the body.

The program is achieved through the following technical measures:
The filler for spine in the first example includes a catheter and a catheter holder for fastening the catheter, there is a guide pin inside of the catheter, and the guide pin is fastened on the guide pin holder, a sealed or ventilate feed bag made of soft nonmetal materials according to biocompatibility or absorbefacient materials is set at the end of the catheter, the feature of the program lies in that a outer thimble is set at the outer side of the catheter, the outer thimble is fixed on the outer thimble holder, a dismountable structure connects the outer thimble holder with the catheter holder, a reducing cone of tapered outer thimble at the open end of the said outer thimble connects to a little diameter outer thimble; the said catheter has a reducing cone corresponding to reducing cone of outer thimble at the reducing cone of outer thimble, and the reducing cone of catheter connects to a little diameter catheter; the said feed bag is set in the catheter through its open end, lies in between outer thimble and catheter and fastened tightly at the outer wall of catheter by outer thimble. The specific feature of the program also includes: the length of the said little diameter catheter is 1-40mm, that of the said little diameter outer thimble is 0-10mm, and that of the said feed bag is between 10-50mm. The said feed bag is a monolayer, and a protective layer for feed bag is at the outer wall of the said feed bag, the dismountable connection between the said outer thimble holder and catheter holder is achieved through a thread.

The embodiment is achieved through the following technical measures:
The filler for spine in the second embodiment includes a catheter and a catheter holder for fastening the catheter, there is a guide pin inside of the catheter, and the guide pin is fastened on the guide pin holder, a sealed or ventilate feed bag made of soft nonmetal materials according to biocompatibility or absorbefacient materials is set at the end of the catheter, the feature of the program lies in that a outer thimble is set at the outer side of the catheter, the outer thimble is fixed on the outer thimble holder, a dismountable structure connects the outer thimble holder with catheter holder, a inside convex plate is set at the open end of outer thimble; the said catheter has a reducing cone corresponding to inside convex plate of outer thimble at the inside convex plate of outer thimble, and the reducing cone of catheter connects to a little diameter catheter; the said feed bag is set in the catheter through its open end, lies in between outer thimble and catheter and fastened tightly at the outer wall of catheter by outer thimble. Other specific features of the program includes: the length of the said little diameter catheter is 1-40mm, that of the said little diameter outer thimble is 0.5-10mm, and that of the said feed bag is between 10-50mm. The said feed bag is a monolayer, and a protective layer for feed bag is at the outer wall of the said feed bag, the dismountable connection between the said outer thimble holder and catheter holder is achieved through a thread.

The beneficial effects of the fillers can be recognized by the above narration, in the two structures, the said feed bag is set at the outer wall of the catheter by outer thimble, so the born materials such as ferroconcrete can be injected into the feed bag by catheter to support the spine of the patient. when the surgery finished, the catheter and outer thimble could be drawn out and useless foreign matter need not leave in the body. The difference of the two fillers only lies in the structure limits the feed bag not to slip freely. In the first example, the structure adopted is a reducing cone of a tapered outer thimble connects to a little diameter outer thimble at the open end of the outer thimble, and the catheter has a reducing cone corresponding to that of outer thimble at the reducing cone of outer thimble, and the reducing cone of catheter connects to a little diameter catheter. In the second embodiment, the structure adopted is a inside convex plate at the open end of the outer thimble, and the catheter has a reducing cone corresponding to the inside convex plate at the inside convex plate of outer thimble, and reducing cone of catheter connects a little diameter catheter. The two programs adopt the changing diameter of feed bag when moving, to limit the feed bag not to move freely. Thus, the present invention has substantive features and progress compared with existing technologies, it is also obvious for its beneficial effects of implementation.

### Description of the drawings:

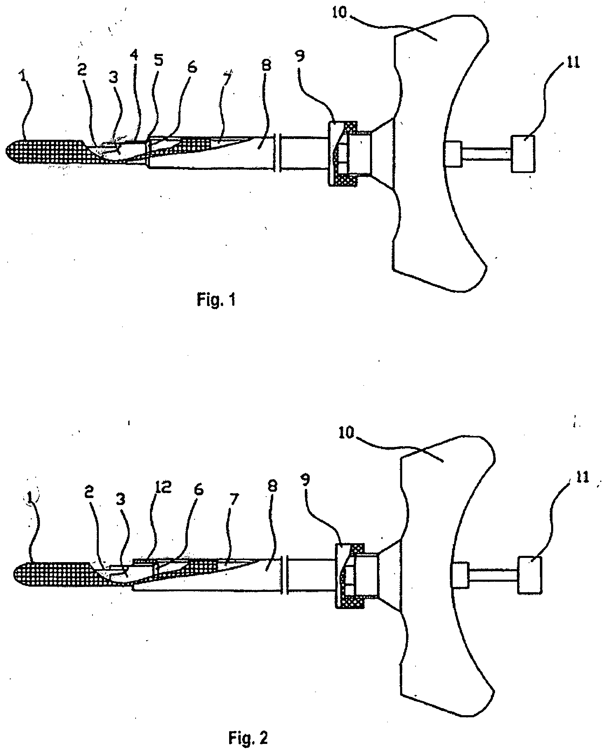

FIG.1 is the partly sectional structure view of embodiment of the invention.

Where, 1 is feed bag, 2 is guide pin, 3 is little diameter catheter, 4 is little outer thimble, 5 is reducing cone of outer thimble, 6 is reducing cone of catheter, 7 is catheter, 8 is outer thimble, 9 is outer thimble holder, 10 is catheter holder, 11 is guide pin holder, 12 is inside convex plate of outer thimble.

### Detailed description of the invention

In order to clearly explain the technical features of the program, the following will describe the programs by two embodiments and their drawings.

It can be seen from the FIG 1, the filler for spine of the example includes a catheter 7 and a catheter holder 10 for fastening catheter 7. there is a guide pin 2 inside of the catheter 7, and the guide pin 2 is fastened on the guide pin holder 11, a sealed or ventilate feed bag 1 made of soft nonmetal materials according to biocompatibility or absorbefacient materials is set at the end of the catheter 2, a outer thimble 8 is set at the outer side of the said catheter 7 in this embodiment, and the outer thimble 8 is fixed on the outer thimble holder 9, a dismountable structure connects the outer thimble holder 9 and catheter holder 10, the thread connection is adopted in this embodiment. a reducing cone of tapered outer thimble 5 at the open end of the said outer thimble 8 connects to a little diameter outer thimble 4; the length of the little diameter outer thimble 4 is 0-10mm;the said catheter 7 has a reducing cone of catheter 6 corresponding to reducing cone of outer thimble 5 at the reducing cone of outer thimble 5, and the reducing cone of catheter 6 connects to a little diameter catheter 3, the length of the little diameter catheter 3 is 1-40mm; In this embodiment, the length of the little diameter outer thimble 4 is 3mm, and that of the little diameter catheter 3 is 10mm. The said feed bag 1 is set in the catheter 7 through its open end, lies in between outer thimble 8 and catheter 7 and fastened tightly at the outer wall of catheter 7 by outer thimble 8. The length of the feed bag 1 is between 10-50mm. The said feed bag 1 can be a monolayer or a protective layer is added at the out wall of the feed bag 1.

It can be seen from FIG 2, the filler for spine in the embodiment includes a catheter 7 and a catheter holder 10 for fastening the catheter 7, there is a guide pin 2 inside of the catheter 7, and the guide pin 2 is fastened on the guide pin holder 11, a sealed or ventilate feed bag 1 made of soft nonmetal materials according to biocompatibility or absorbefacient materials is set at the end of the catheter 2, a outer thimble 8 is set at the outer side of the said catheter 7 in this embodiment, the outer thimble 8 is fixed on the outer thimble holder 9, a dismountable structure connects the outer thimble holder 9 and the catheter holder 8, the thread connection is adopted in this embodiment. A inside convex plate 12 is set at the open end of outer thimble 8, the length of the inside convex plate 12 is 0.5-10mm; the said catheter 7 has a reducing cone 6 corresponding to inside convex plate of outer thimble 12 at the inside convex plate of outer thimble 12, and the reducing cone 6 of catheter connects to a little diameter catheter 3; the length of the little catheter 3 is 1-40mm. In this embodiment, the length of the inside convex plate 12 of outer thimble is 5mm, and that of the little diameter of catheter 3 is 15mm. The said feed bag 1 is set in the catheter 7 through its open end, lies in between outer thimble 8 and catheter 7 and fastened tightly at the outer wall of catheter 7 by outer thimble 8, the length of feed bag 1 is between 10-50mm. The said feed bag 1 can be a monolayer or a protective layer is added at the out wall of the feed bag1.

## Claims

1. A filler for spine includes a catheter (7) and a catheter holder (10) for fastening the catheter, wherein there is a guide pin (2) inside of the catheter, and the guide pin is fixed on the guide pin holder (11), a sealed or ventilate feed bag (1) made of soft nonmetal materials according to biocompatibility or absorbefacient materials is set at the end of the catheter (7), wherein an outer thimble (8) is set at the outer side of the said catheter, the outer thimble is fixed on the outer thimble holder (9), a dismountable structure connects the outer thimble holder and catheter holder (10),
**characterized in that**
an inside convex plate (12) of outer thimble is set at the open end of the said outer thimble; wherein the catheter has a reducing cone corresponding to the inside convex plate of outer thimble at the inside convex plate of outer thimble, and the reducing cone of catheter (6) connects to a little diameter catheter (3); the feed bag (1) is set in the catheter through its open end, lies in between outer thimble (8) and catheter (7) and fastened tightly at the outer wall of catheter by outer thimble.

2. The filler for spine of claim 1, wherein the length of the said little diameter catheter (3) is 1-40mm.

3. The filler for spine of claim 1 or 2, wherein the length of the said little diameter outer thimble (4) is 0.5-10mm.

4. The filler for spine of claim 1, wherein the length of the said feed bag (1) is 10-50mm.

5. The filler for spine of claim 1, wherein the said feed bag (1) is a monolayer.

6. The filler for spine of claim 1, wherein the said feed bag (1) has a protective layer at its outside wall.

7. The filler for spine of claim 1, wherein the dismountable connection between the said outer thimble holder (9) and the said catheter holder (10) is a thread one.

## Patentansprüche

1. Wirbelfüllstoff, enthaltend einen Katheter (7) und eine Katheterhalterung (10) zum Befestigen des Katheters, wobei ein Führungsstift (2) im Inneren des Katheters vorhanden ist und der Führungsstift an der Führungsstifthalterung (11) fixiert ist, ein versiegelter oder belüfteter Beutel (1), der aus weichen Nicht-Metallmaterialien je nach Biokompatibilität oder absorptionsfördernden Materialien hergestellt ist, der am Ende des Katheters (7) angeordnet ist, wobei eine äußere Kausche (8) an der Außenseite des Katheters angeordnet ist, wobei die äußere Kausche an der äußeren Kauschenhalterung (9) fixiert ist, eine demontierbare Struktur, die die äußere Kauschenhalterung und die Katheterhaltung (10) verbindet,
**dadurch gekennzeichnet, dass**
eine konvexe Innenplatte (12) der äußeren Kausche am offenen Ende der äußeren Kausche angeordnet ist; wobei der Katheter einen reduzierenden Kegel entsprechend der konvexen Innenplatte der äußeren Kausche aufweist und der reduzierende Kegel des Katheters (6) mit einem Katheter (3) geringen Durchmessers verbunden ist; wobei der Beutel (1) im Katheter durch dessen offenes Ende angeordnet wird, zwischen der äußeren Kausche (8) und dem Katheter (7) liegt und durch die äußere Kausche eng an der Außenwand des Katheters befestigt ist.

2. Wirbelfüllstoff nach Anspruch 1, wobei die Länge des Katheters (3) geringen Durchmessers 1 bis 40 mm ist.

3. Wirbelfüllstoff nach Anspruch 1 oder 2, wobei die Länge der äußeren Kausche (4) geringen Durchmessers 0,5 bis 10 mm ist.

4. Wirbelfüllstoff nach Anspruch 1, wobei die Länge des Beutels (1) 10 bis 50 mm ist.

5. Wirbelfüllstoff nach Anspruch 1, wobei der Beutel (1) eine Monoschicht ist.

6. Wirbelfüllstoff nach Anspruch 1, wobei der Beutel (1) eine Schutzschicht an seiner Außenwand aufweist.

7. Wirbelfüllstoff nach Anspruch 1, wobei die demontierbare Verbindung zwischen der äußeren Kauschenhalterung (9) und der Katheterhalterung (10) ein Faden ist.

## Revendications

1. Dispositif de remplissage de colonne vertébrale comprenant un cathéter (7) et un support de cathéter (10) pour fixer le cathéter, dans lequel il y a une broche de guidage (2) à l'intérieur du cathéter, et la broche de guidage est fixée sur le support de broche de guidage (11), une poche d'alimentation étanche ou ventilée (1) fabriquée en matériaux non-métalliques souples conformément à leur biocompatibilité ou en matériaux absorbants est disposée à l'extrémité du cathéter (7), dans lequel un manchon extérieur (8) est disposé sur le côté extérieur dudit cathéter, le manchon extérieur est fixé sur le support de manchon extérieur (9), une structure démontable relie le support de manchon extérieur au support de cathéter (10),
**caractérisé en ce que**
une plaque convexe intérieure (12) du manchon extérieur est disposée au niveau de l'extrémité ouverte dudit manchon extérieur ; dans lequel le cathéter a un cône réducteur correspondant à la plaque convexe intérieure du manchon extérieur au niveau de la plaque convexe intérieure du manchon extérieur, et le cône réducteur de cathéter (6) se raccorde à un cathéter de petit diamètre (3) ; la poche d'alimentation (1) est disposée dans le cathéter à travers son extrémité ouverte, se situe entre le manchon extérieur (8) et le cathéter (7) et est solidement fixée à la paroi extérieure du cathéter par le manchon extérieur.

2. Dispositif de remplissage de colonne vertébrale selon la revendication 1, dans lequel la longueur dudit cathéter de petit diamètre (3) est de 1 à 40 mm.

3. Dispositif de remplissage de colonne vertébrale selon la revendication 1 ou 2, dans lequel la longueur dudit manchon extérieur de petit diamètre (4) est de 0,5 à 10 mm.

4. Dispositif de remplissage de colonne vertébrale selon la revendication 1, dans lequel la longueur de ladite poche d'alimentation (1) est de 10 à 50 mm.

5. Dispositif de remplissage de colonne vertébrale selon la revendication 1, dans lequel ladite poche d'alimentation (1) est une monocouche.

6. Dispositif de remplissage de colonne vertébrale selon la revendication 1, dans lequel ladite poche d'alimentation (1) comporte une couche de protection au niveau de sa paroi extérieure.

7. Dispositif de remplissage de colonne vertébrale selon la revendication 1, dans lequel le raccord démontable entre ledit support de manchon extérieur (9) et ledit support de cathéter (10) est un raccord fileté.
